# EUROPÄISCHE PATENTSCHRIFT

(11) **EP 4 326 393 B1**
(45) Veröffentlichungstag und Bekanntmachung des Hinweises auf die Patenterteilung: **19.02.2025**
(21) Anmeldenummer: 22720048.2
(22) Anmeldetag: 19.04.2022
(51) Int. Cl.: A61N 5/06

(54) **VORRICHTUNG ZUR STIMULATION DER BIOSYNTHESE VON VITAMIN D3**
DEVICE FOR STIMULATING THE BIOSYNTHESIS OF VITAMIN D3
DISPOSITIF DE STIMULATION DE LA BIOSYNTHÈSE DE LA VITAMINE D3

(30) Priorität: 21.04.2021 CH 4252021
(43) Veröffentlichungstag der Anmeldung: 28.02.2024
(73) Patentinhaber: JK-Holding GmbH, 53578 Windhagen (DE)
(72) Erfinder: GERSTENMEIER, Jürgen, 56567 Neuwied (DE)
(74) Vertreter: IPrime Rentsch Kaelin AG
(86) Internationale Anmeldenummer: PCT/IB2022/053653
(87) Internationale Veröffentlichungsnummer: WO 2022/224140

(56) Entgegenhaltungen:
- WO-A1-2017/180054
- KR-A- 20150 124 016
- US-A1- 2017 348 543

## Beschreibung

Die vorliegende Erfindung, welche in den unabhängigen Ansprüchen 1, 15 und 23 definiert ist, betrifft eine Vorrichtung zur Stimulation der Biosynthese vor Vitamin D3, sowie ein Computerprogrammprodukt zur Steuerung einer solchen.

### Technologischer Hintergrund

Vitamine sind für einen Organismus essenzielle Stoffe, die an zahlreichen Reaktionen des Stoffwechsels teilhaben und einen Einfluss auf das Immunsystem haben. Unter den Vitaminen nimmt das Vitamin D eine besondere Rolle ein. Vitamin D ist eine lipophile Verbindung, die von den meisten Wirbeltieren durch ultraviolette Lichtstrahlung auf die Haut synthetisiert werden kann. Dafür verantwortlich ist vor allem der UV-B-Anteil im Sonnenlicht, also Lichtstrahlung der Wellenlängen zwischen 280 nm und 315 nm.

In vielen Breitengraden oder bei der Ausübung von Tätigkeiten, die hauptsächlich innerhalb von Gebäuden oder nachts stattfinden, kann es erforderlich sein, Vitamin D3 zu supplementieren. Neben Vitamin-D-haltigen Nahrungsmitteln, kann man Vitamin D auch als Nahrungsmittelergänzung zu sich nehmen. Wichtig ist es, dass bei der Einnahme von Vitamin-D-Tabletten auch ein entsprechendes Fett oder Öl eingenommen wird, um die Aufnahme zu erleichtern. Grundsätzlich kann in Umgebungen, in denen es nicht möglich ist, sich über eine tägliche Exposition einer bestimmten Menge an Sonnenlicht, oder über entsprechende Supplemente den Vitamin-D-Gehalt auf ausreichendem Pegel zu halten, möglich, über eine Lichtbestrahlung die Biosynthese durch die Haut zu nutzen, so wie es mit einer Sonnenexposition geschehen würde. So können z.B. Solariumbesuche von bereits kurzer Bestrahlungsdauer ausreichen, um die körpereigene Biosynthese von Vitamin D3 auf einem ausreichenden Grad zu halten. Vermehrt treten Produkte auf den Markt, die Leuchtmittel für den täglichen Bedarf bewerben, die die Biosynthese von Vitamin D3 zusätzlich unterstützen sollen. Dabei gilt es aber zu beachten, dass gerade die einschlägige UV-Strahlung im Wellenlängenbereich von zwischen 280 nm und 315 nm die Augen schädigen kann. UV-Strahlung der Typen A und B werden in der Regel von der Augenlinse absorbiert, sodass mit der Zeit Trübungseffekte auftreten können und in Katarakten enden, die operativ behandelt werden müssen. Zusätzlich besteht ein gewisses Krebsrisiko durch die besagte UV-Strahlung, sodass es zu bevorzugen ist, die Exposition kontrolliert und nicht für lange Zeiträume auszuführen.

Vor allem Berufsgruppen, die regelmässig Nachtarbeit leisten müssen, oder Menschen, die an Orten leben, an denen die tägliche Sonneneinstrahlung insbesondere in den Wintermonaten sehr gering bis gänzlich abwesend ist, profitieren stark von Leuchtmitteln, welche die körpereigene und natürliche Biosynthese von Vitamin D3 ermöglichen. Im Pandemiejahr 2020 hat eine wissenschaftliche Studie zudem starke Indizien dafür gefunden, dass UV-B-Strahlung einen durch die Vitamin-D3-Synthese gestützten Schutzeffekt gegen einen schweren Verlauf von Covid-19 haben kann (Evidence of protective role of Ultraviolet-B (UVB) radiation in reducing COVID-19 deaths [Moozhipurath, R.K., Kraft, L. und Skiera, B., Sei Rep 10, 17705 [2020]).

Im Sinne der vorliegenden Erfindung, und ohne an diese Theorie gebunden zu sein, wird für die Bestrahlung eine Biosynthese von Vitamin D3 und ihren entsprechenden Derivaten angenommen, die mit ultraviolettem Licht in den Wellenlängen von zwischen 290 bis 315 nm, also Wellenlängen im UV-B-Bereich mit einer Energie von mindestens der halben minimalen Erythemdosis (MED) synthetisiert werden kann. Dazu wird 7-Dehydrocholesterol durch eine fotochemisch induzierte Reaktion des B-Rings aufgebrochen und Prävitamin-D3 gebildet. Dieses Prävitamin D3 ist instabil und reagiert zu Vitamin D3. Im Blut wird es an das Vitamin-D-bindende Protein gebunden und zur Leber transportiert, wo es zu Calcitriol hydroxyliert wird. Die erforderliche Vorstufe, also das 7-Dehydrocholesterol wird vom Körper selbst hergestellt.

Zusammenfassend ist festzuhalten, dass es einen Bedarf nach Vorrichtungen gibt, welche die körpereigene Biosynthese von Vitamin D3 stimulieren oder mindestens unterstützen können. Diese Vorrichtungen sollen in der Anwendung möglichst wenig schädliche Nebeneffekte aufweisen. KR 2015 0124016 A offenbart eine Vorrichtung gemäß dem Oberbegriff von Anspruch 1.

### Darstellung der Erfindung

Es ist somit eine Aufgabe der vorliegenden Erfindung, eine Vorrichtung der eingangs genannter Art bereitzustellen, die mindestens einen Nachteil des Bekannten überwindet. Insbesondere soll eine derartige Vorrichtung bereitgestellt werden, die sicher im Gebrauch ist und einen messbaren Effekt auf den Vitamin-D3-Gehalt im Blut aufweisen kann. Es ist eine weitere Aufgabe der vorliegenden Erfindung, ein Computerprogrammprodukt bereitzustellen, welches zur Steuerung einer derartigen Vorrichtung geeignet ist und einen sicheren Betrieb einer solchen Vorrichtung ermöglicht.

Es ist eine weitere, besondere Aufgabe der vorliegenden Erfindung, eine derartige Vorrichtung und ein entsprechendes Computerprogrammprodukt bereitzustellen, welches eine Schädigung der Augenlinsen durch ihre Nutzung hemmt oder im Wesentlichen verhindert.

Mindestens eine dieser Aufgaben wurde mit einer Vorrichtung und einem Computerprogrammprodukt gemäss kennzeichnendem Teil der unabhängigen Ansprüche gelöst.

Ein Aspekt der vorliegenden Erfindung betrifft eine Vorrichtung zur Stimulation der Biosynthese von Vitamin D3. Die Vorrichtung umfasst mindestens ein erstes Leuchtmittel zur Emission von Lichtstrahlung umfassend Wellenlängen in einem Bereich von zwischen 280 nm und 315 nm. Die Vorrichtung umfasst weiter eine Sensoreinheit zur Erkennung eines Bestrahlungsbereichs. Sie umfasst weiter eine Richteinheit zur Ausrichtung des mindestens einen Leuchtmittels auf den Bestrahlungsbereich. Zudem umfasst die erfindungsgemässe Vorrichtung eine Steuereinheit, welche ausgelegt ist, den Bestrahlungsbereich zu identifizieren, und eine Emission von Lichtstrahlung umfassend Wellenlängen in einem Bereich von zwischen 280 und 315 nm durch das mindestens eine Leuchtmittel auszulösen.

Ein Vorteil der erfindungsgemässen Vorrichtung kann sein, dass mittels der Sensoreinheit sichergestellt werden kann, dass die Emission von Lichtstrahlung nur dann eingesetzt wird, wenn ein Bestrahlungsbereich erkannt wird, der für die Bestrahlung sicher ist. Dies kann z.B. anhand von vordefinierten Kriterien festgelegt werden.

Es wurde überraschend gefunden, dass mit einer erfindungsgemässen Vorrichtung ein Bestrahlungsbereich, z.B. ein Hautbereich eines Menschen, mit ausreichender Lichtstrahlung umfassend Wellenlängen insbesondere im UV-B-Bereich bestrahlt werden kann, um die körpereigene Bildung von Vitamin D zu stimulieren oder mindestens zu unterstützen und dabei unterhalb einer minimalen Erythemdosis für einen hellhäutigen Menschen zu bleiben.

Im Sinne der vorliegenden Erfindung ist als minimale Erythremdosis der Schwellenwert zu verstehen, über dem ein Erythem, also Sonnenrandeffekt auf der Haut beobachtet werden kann. Die minimale Erythemdosis ist unterschiedlich für verschiedene Hauttypen, und unterliegt auch individuellen Schwankungen. Für die vorliegende Erfindung kann zum Beispiel als Referenz angenommen werden, dass eine Bestrahlungsdosis von ca. 12.5 mJ/cm² für einen Zeitraum von zwischen 10 bis 15 Minuten ausreicht um bei einem Menschen vom Hauttyp 2 ausreichen kann, um eine Biosynthese von Vitamin D₃ zu stimulieren. Im Sinne der vorliegenden Erfindung kann der Hauttyp als gemäss Einteilung nach Fitzpatrick verstanden werden in folgende Typen: I (sehr hell), II (hell), III (mittelhell), IV (bräunlich, olivfarben), V (hellbraun, dunkel) und VI (dunkelbraun bis schwarz) [T. B. Fitzpatrick: Ultraviolet-induced pigmentary changes: Benefits and hazards. In: Therapeutic Photomedicine. (= Current Problems in Dermatology. Band 15). Karger, 1986, S. 25-38].

In einer besonderen Ausführungsform umfasst das Leuchtmittel mindestens eine LED. Im Sinne der vorliegenden Erfindung ist ein Leuchtmittel zur Emission von Lichtstrahlung umfassend Wellenlängen in einem Bereich von zwischen 280 nm und 315 nm zu verstehen als ein Leuchtmittel, welches mindestens einen Teilbereich dieses Spektrums abdeckt. Geeignet sind z.B. auch UV-LEDs mit einem Spektrum von zwischen 280 und 315 nm, ebenso geeignet sind LEDs, welche einen Peak im besagten Spektrumsbereich aufweisen. Geeignete Leuchtmittel können von einem Fachmann ermittelt werden.

LEDs bieten einen weiteren Vorteil: so wurde insbesondere überraschend gefunden, dass LEDs sogar noch effizienter zur Stimulation der Biosynthese von Vitamin D3 eingesetzt werden können als natürliches Sonnenlicht. (Kalajian, T.A., Aldoukhi, A., Veronikis, A.J. et al. Ultraviolet B Light Emitting Diodes (LEDs) Are More Efficient and Effective in Producing Vitamin D3 in Human Skin Compared to Natural Sunlight. Sci Rep 7, 11489 (2017)).

Besonders bevorzugt ist das Leuchtmittel ausgelegt zur Emission von Lichtstrahlung umfassend Wellenlängen in einem Bereich von zwischen 290 nm und 300 nm.

Im Sinne der vorliegenden Erfindung kann z.B. unter einer Richteinheit eine mechanische Anordnung verstanden werden, welche geeignet ist, das mindestens eine erste Leuchtmittel und/oder mindestens die Lichtstrahlung des mindestens einen ersten Leuchtmittels auf einen Bestrahlungsbereich zu richten. Dies kann z.B. dadurch bewerkstelligt werden, dass eine Richteinheit auf mindestens einer Achse bewegbar und/oder schwenkbar ist. Bevorzugt ist die Richteinheit auf mindestens zwei Achsen bewegbar und/oder schwenkbar, sodass bevorzugt ein Kegelbereich, ausgehend vom mindestens einen ersten Leuchtmittel, von der Richteinheit abgefahren werden kann, sodass innerhalb dieses Kegelbereichs eine Mehrzahl an Bestrahlungsbereichen erfasst werden kann.

In einer besonderen Ausführungsform ist die Richteinheit ausgelegt das mindestens eine Leuchtmittel in einem Winkelbereich von 180° auszurichten.

In einer besonderen Ausführungsform umfasst die Steuereinheit mindestens einen Prozessor, der ausgelegt ist, ein Computerprogramm auszuführen, welches geeignet ist, um Daten, die von der Sensoreinheit empfangen werden, auszuwerten. Diese Auswertung ist z.B. ausgelegt, um einen bestimmten Bestrahlungsbereich zu identifizieren. Die Steuereinheit kann ausgelegt sein, um entsprechende Prozesse auszuführen, sobald ein Bestrahlungsbereich identifiziert wurde. Diese Prozesse können z.B. eine Emission von Lichtstrahlung in den besagten Wellenlängen umfassen, oder ein Abschalten einer Emission von Lichtstrahlung in den besagten Wellenlängen. Weiter können diese Prozesse ausgelegt sein, um eine Ausrichtung durch die Richteinheit zu veranlassen. Geschieht eine Erkennung eines Bestrahlungsbereichs kontinuierlich, so kann die Steuereinheit z.B. ausgelegt sein, um der Richteinheit Anweisungen zu geben, einen bestimmten Verlauf abzufahren und innerhalb eines Kegels z.B., den die Richteinheit erfassen kann, einen geeigneten Bestrahlungsbereich zu finden. Ist ein solcher einmal identifiziert, kann die Steuereinheit anschliessend eine Emission von Lichtstrahlung in den besagten Wellenlängen auslösen.

In einer besonderen Ausführungsform umfasst die Sensoreinheit einen optischen Sensor. Insbesondere ist der optische Sensor eine Kamera. Grundsätzlich sind sämtliche Sensorenelemente für eine erfindungsgemässe Sensoreinheit geeignet, welche in der Lage sind, über eine gewisse Distanz einen Bestrahlungsbereich zu erkennen. Es hat sich gezeigt, dass optische Sensoren besonders geeignet sind. Die Sensoreinheit kann konkret einen Sensor umfassen, z.B. eine Kamera. Sie kann zudem weitere Sensoren umfassen, wie z.B. einen Entfernungsmesser. Geeignete Entfernungsmesser können z.B. auf der Basis von Laserentfernungsmessern vorgesehen sein, welche in der Lage sind, einen Laserstrahl abzusenden und dadurch akkurat eine Distanz zu einem Objekt zu messen. Dies hat insbesondere den Vorteil, dass das Gerät über die Steuerung präzise eine Intensität für das Leuchtmittel bestimmen kann, welches der Distanz des Bestrahlungsbereichs zum Leuchtmittel Rechnung trägt. So kann weiter die optimale Stimulation der Biosynthese von Vitamin D3 ermöglicht werden und sichergestellt werden, dass eine Lichtstrahlung so angewandt wird, dass sie unterhalb der kritischen Erythemdosis bleibt.

Der optische Sensor kann auch ausgelegt sein, um Infrarotbilder zu erfassen. Ein solchermassen ausgestatteter Sensor kann z.B. die Wirkung einer Lichtstrahlung auf einen Bestrahlungsbereich nachverfolgen. Dies kann weiter das Einhalten der minimalen Erythemdosis unterstützen. Zudem kann es anhand der thermischen Gradienten ermöglicht werden, einen bestimmten Energiewert zu ermitteln, der auf den Bestrahlungsbereich beaufschlagt wurde. Aus diesem kann dann z.B. die Verabreichung einer ausreichenden Dosis der genannten Lichtstrahlung berechnet werden.

In einer besonderen Ausführungsform umfasst die Vorrichtung eine Mehrzahl an Sensoreinheiten jeweils mit einem oder mehreren Sensoren, insbesondere optischen Sensoren.

In einer besonderen Ausführungsform ist die Steuereinheit ausgelegt, um eine Ausrichtung des ersten Leuchtmittels durch die Richteinheit anhand eines identifizierten Bestrahlungsbereichs zu steuern. In dieser Ausführungsform kann z.B. eine Sensoreinheit mit einem optischen Sensor ausgestattet werden, der einen bestimmten Bereich abdeckt. Der Bereich kann mittels eines Weitwinkelobjektivs weit gefasst sein. Wird innerhalb dieses Bereichs ein geeigneter Bestrahlungsbereich von der Sensoreinheit erkannt und von der Steuereinheit identifiziert, so kann die Steuereinheit z.B. die Richteinheit veranlassen, das Leuchtmittel auf diesen Bereich auszurichten. Dazu kann die Steuereinheit ausgelegt sein, um den Bestrahlungsbereich anhand eines Vektors im Bildbereich des optischen Sensors festzulegen und die Richteinheit zur Ausrichtung des Leuchtmittels anhand dieses Vektors zu veranlassen.

In einer besonderen Ausführungsform umfasst die erfindungsgemässe Vorrichtung eine Sammeleinheit zur Erzeugung eines gerichteten Strahlenbündels aus der Emission von Lichtstrahlung durch das erste Leuchtmittel. Die Sammeleinheit ist bevorzugt ausgelegt, um einen begrenzten Bestrahlungsbereich mit der Lichtstrahlung zu beaufschlagen. Dazu verfügt die Sammeleinheit z.B. über Elemente, welche es ermöglichen, die Lichtstrahlung über die Distanz zum Bestrahlungsbereich innerhalb eines gewissen Raumes zu halten, so dass der Bestrahlungsbereich möglichst präzise definiert ist.

In einer besonderen Ausführungsform umfasst die Sammeleinheit mindestens einen Reflektor. So kann z.B. als Leuchtmittel eine LED verwendet werden und ein entsprechender um die LED angeordneter Reflektor vorgesehen sein, welche die Lichtstrahlung, die von der LED emittiert wird, auf einen bestimmten Bestrahlungsbereich richtet. Besonders bevorzugt ist eine Reflektoreinheit vorgesehen, welche verstellbar ist. Eine verstellbare Reflektoreinheit kann z.B. mit einer Mehrzahl an aktivierbaren Reflektorebenen ermöglicht werden, womit z.B. einer bestimmten Distanz oder einem bestimmten Winkel des Bestrahlungsbereichs relativ zum Leuchtmittel Rechnung getragen werden kann.

In einer besonderen Ausführungsform umfasst die Sammeleinheit eine Linse und/oder einen Kollimator. Besonders bevorzugt umfasst die Sammeleinheit einen Kollimator mit einer Sammellinse zur Erzeugung eines gerichteten Strahlenbündels aus der Emission von Lichtstrahlung durch das erste Leuchtmittel. Je nach Ausgestaltung des Gerätes, Endgrösse und Platzierung relativ zum Bestrahlungsbereich kann von einem Fachmann die Sammeleinheit bei Bedarf anders ausgestaltet werden.

In einer besonderen Ausführungsform umfasst die erfindungsgemässe Vorrichtung ein zweites Leuchtmittel zur Emission von Lichtstrahlung umfassend Wellenlängen im sichtbaren Bereich. Besonders bevorzugt ist das zweite Leuchtmittel ausgelegt, um Lichtstrahlung umfassend Wellenlängen in einem Bereich von zwischen 380 nm und 780 nm zu emittieren. Durch die Ausstrahlung von sichtbarem Licht kann der Betrieb der erfindungsgemässen Vorrichtung idealerweise für einen Menschen verfolgt werden. So kann bevorzugt dieses zweite Leuchtmittel ausgelegt sein, um denselben Bestrahlungsbereich zu bestrahlen, wie das erste Leuchtmittel. Durch den erzeugten «Lichtkegel» sieht der Anwender, dass ein bestimmter Bestrahlungsbereich gerade mit der entsprechenden Lichtstrahlung beaufschlagt wird. So kann z.B. der Anwender angeregt werden, den Bereich für die Dauer der Bestrahlung stillzuhalten, und so sicherzustellen, dass das Gerät die definierte und optimale Strahlungsmenge abgeben kann, um die beabsichtigte Stimulation der Biosynthese von Vitamin D3 optimal zu vollführen.

In einer besonderen Ausführungsform umfasst die erfindungsgemässe Vorrichtung ein drittes Leuchtmittel zur Emission von Lichtstrahlung umfassend Wellenlängen im nahen Infrarotbereich. Besonders bevorzugt bewegen sich diese Wellenlängen in einem Bereich von zwischen 640 nm und 1.800 nm. Ohne an diese Theorie gebunden zu sein, kann eine Beaufschlagung des Bestrahlungsbereichs mit Strahlung im nahen Infrarotbereich die Durchblutung dieses Bereichs verbessern. Nahinfrarotstrahlung wird eingesetzt als Durchblutungsförderer für die tieferen Schichten des Gewebes. Dadurch kann eine Biosynthese von Vitamin D3 noch stärker angeregt werden, was es wiederum ermöglicht, die Dosis an UV-B-Strahlung weiter zu reduzieren.

In einer besonderen Ausführungsform ist die Steuereinheit ausgelegt, ein menschliches Gesicht in einem Bestrahlungsbereich zu identifizieren. Dies kann z.B. dadurch erfolgen, dass ein durch eine Sensoreinheit erkannter Bestrahlungsbereich mit einer Datenbank verglichen wird. Verfahren zur Gesichtserkennung sind inzwischen bekannt und werden breit eingesetzt. Moderne Kameras, z.B. wie sie auch in gegenwärtigen Mobiltelefonen verbaut werden, verfügen über eine Gesichtserkennung, um die entsprechende Schärfe der Bildaufnahme zu unterstützen. Im Sinne der vorliegenden Erfindung reicht eine Gesichtserkennung, welche die Anwesenheit eines Gesichts erkennt und nicht zwingend das Gesicht einer bestimmten Person zuordnen kann. Dennoch wäre es denkbar, die Gesichtserkennung zu nutzen, um eine bestimmte Person zu identifizieren. Dies kann es z.B. ermöglichen, mit der erfindungsgemässen Vorrichtung eine bestimmte Dosis über einen gewissen Zeitraum an eine Person abzugeben. Das Gerät kann somit feststellen, dass die besagte Person innerhalb eines gewissen Zeitraums ausreichend mit der Lichtstrahlung beaufschlagt wurde, um die entsprechende Biosynthese von Vitamin D3 zu stimulieren. Zweidimensionale und dreidimensionale Verfahren zur Gesichtserkennung, welche für die Nutzung in der vorliegenden Erfindung geeignet sind, sind dem Fachmann bekannt und können bei Bedarf an die Anforderungen angepasst werden, z.B. je nachdem, ob eine blosse Erkennung, ob ein Gesicht da ist, oder bereits eine Zuordnung eines Gesichts an eine bestimmte Person erwünscht ist.

In einer besonderen Ausführungsform ist die Steuereinheit ausgelegt, um ein Gesicht eines minderjährigen Menschen zu erkennen, und diesen Menschen von der Bestrahlung auszuschliessen. In einer besonderen Ausfügungsform ist die Steuereinheit ausgelegt, um ein erkanntes Gesicht mit einem Register abzugleichen, welches für die Bestrahlung zugelassen ist. Somit kann sichergestellt werden, dass nur zugelassene und/oder berechtigte Benutzer die erfindungsgemässe Vorrichtung verwenden.

In einer besonderen Ausführungsform ist die Steuereinheit ausgelegt, um einen vordefinierten Bestrahlungsbereich zu identifizieren. So kann z.B. ein bereits bestrahlter Bestrahlungsbereich als vordefinierter Bestrahlungsbereich verstanden werden, sodass die Steuereinheit einen bereits bestrahlten Bestrahlungsbereich zu identifizieren vermag und bei einer zweiten Bestrahlung innerhalb eines bestimmten Zeitintervalls sich für einen anderen Bestrahlungsbereich entscheidet. Somit kann sichergestellt werden, dass eine tägliche Dosis an UV-B-Strahlung auf einen bestimmten Bestrahlungsbereich nicht überschritten wird. Ebenfalls wäre es denkbar, dass die Steuereinheit ausgelegt ist, um vordefinierte Bestrahlungsbereiche zu erkennen, die von einer Bestrahlung ausgeschlossen sind. So können z.B. Bestrahlungsbereiche, welche Hautbereiche sind, die Narbengewebe, Wundgewebe, Schorf, Tätowierungen, Hautveränderungen, wie z.B. Nävii und dergleichen, enthalten, von der Bestrahlung auszuschliessen sind. Solche vordefinierten Bestrahlungsbereiche können in einem Speicher hinterlegt sein, oder sie können aus dem identifizierten Bestrahlungsbereich berechnet werden, ähnlich wie das bei einer Gesichtserkennung geschieht, indem einzelne Objekte zweidimensional oder dreidimensional erfasst werden und dann anhand algorithmischer Methoden identifiziert werden.

In einer besonderen Ausführungsform umfasst die erfindungsgemässe Vorrichtung eine Kommunikationseinheit zum Austausch von Daten. Besonders bevorzugt umfasst die Kommunikationseinheit mindestens eine Schnittstelle zur Datenübertragung mit einem Computergerät, wozu auch ein modernes Mobiltelefon gezählt werden kann. Diese Schnittstelle kann geeignet sein, eine kabellose Datenübertragung zu ermöglichen, z.B. mittels eines WiFi-Protokolls oder eines Bluetooth^{®}-Protokolls. Die Schnittstelle kann allerdings auch geeignet sein, um mittels einer Steck- und Kabelverbindung auf der Basis eines USB-Protokolls eine Datenübertragung sicherzustellen. Durch eine Kommunikationseinheit ist es z.B. möglich, eine Auswertung einer Operation durch die erfindungsgemässe Vorrichtung auf einem Drittgerät zu überwachen oder zu konfigurieren. So kann z.B. eine Nutzung vorsehen, dass bestimmte Nutzerdaten, wie z.B. Hauttyp, Alter, Beruf und Lebenswandel, mit einem Profil hinterlegt sind. Das Gerät kann ausgelegt sein, um anhand dieser Daten eine optimale Unterstützung der Biosynthese von Vitamin D3 sicherzustellen. Die Kommunikationseinheit kann auch ausgelegt sein, um z.B. eine Programmierung in Form einer Firmware der Vorrichtung auf den neuesten Stand zu halten. Ebenfalls denkbar ist, dass die Kommunikationseinheit eine Verbindung zu einem maschinenlerngestützten Programm ermöglicht, wodurch z.B. eine Gesichtserkennung ständig verbessert werden kann. Weitere Funktionen wären dann z.B. die Erkennung von anderen von einer Bestrahlung ausgeschlossenen Benutzerbereichen, wie z.B. Muttermalen oder Wunden, wie weiter oben bereits beschrieben.

In einer besonderen Ausführungsform sind das erste Leuchtmittel und/oder das zweite Leuchtmittel und/oder das dritte Leuchtmittel um die Sensoreinheit physisch gekoppelt. Somit können sie durch die Richteinheit gemeinsam auf den Bestrahlungsbereich gerichtet werden. Diese Anordnung ist besonders vorteilhaft, wenn die erfindungsgemässe Vorrichtung eine «Suchen und Finden»-Funktion aufweisen soll, bei der sie aktiv einen geeigneten Bestrahlungsbereich identifiziert. So kann z.B. ein Bewegungssensor in der Sensoreinheit vorgesehen sein, welche erkennt, dass sich ein Mensch in einem Wirkbereich der erfindungsgemässen Vorrichtung befindet. Sodann kann die Sensoreinheit beginnen, bestimmte Bestrahlungsbereiche zu erkennen und durch die Steuereinheit zu identifizieren. Die Steuereinheit kann eine Richteinheit solange dazu veranlassen, innerhalb des Wirkbereichs der Vorrichtung nach einem geeigneten Bestrahlungsbereich zu suchen, bis ein solcher gefunden wird.

In einer besonderen Ausführungsform ist die Richteinheit ausgelegt, um mindestens in zwei Achsen verschwenkbar zu sein. Besonders bevorzugt weist sie mindestens einen rotierbar gelagerten Ständerboden und einen rotierbar gelagerten Werkzeugarm zum Verschwenken des Leuchtmittels auf mindestens zwei Achsen auf. Durch eine solche Ausgestaltung ist es möglich, den Wirkbereich der erfindungsgemässen Vorrichtung auf eine Halbkugel auf der Ebene, auf die die Vorrichtung aufgelegt wird, auszudehnen. Durch die Bewegung auf zwei Achsen kann der gesamte Raum innerhalb dieser Hauptkugel von der Sensoreinheit erfasst werden.

In einer besonderen Ausführungsform ist der Bestrahlungsbereich ein Hautbereich mit einer Fläche von zwischen 40 cm² und 900 cm², insbesondere von zwischen 50 cm² und 600 cm², besonders bevorzugt von ungefähr 400 cm².Weitere Geeignete Bestrahlungsprotokolle kann ein Fachmann zum Beispiel aus der Studie von Kalajian, T.A., Aldoukhi, A., Veronikis, A.J. et al und Moozhipurath, R.K. et al, s.o. entnehmen.

In einer besonderen Ausführungsform ist die Hautfläche dieses Bestrahlungsbereichs durch eine regulierte Sammeleinheit definierbar. Dazu kann die Sammeleinheit z.B. eine verstellbare Blende aufweisen, welche den Kegel des Lichtstrahls entsprechend weiten und beschränken kann, sodass der Bestrahlungsbereich innerhalb gewisser Parameter definiert werden kann.

Mit der erfindungsgemässen Vorrichtung wird eine Vorrichtung bereitgestellt, die es ermöglicht, gezielt und mit einer guten Kontrolle möglicher Nebenwirkungen eine Stimulation der Biosynthese von Vitamin D3 zu ermöglichen.

Ein weiterer Aspekt der vorliegenden Erfindung betrifft ein Computerprogrammprodukt zur Durchführung einer Steuerung einer Vorrichtung zur Stimulation von Biosynthese von Vitamin D3. Besonders bevorzugt wird das Computerprogrammprodukt durch eine Steuereinheit der oben beschriebenen Vorrichtung ausgeführt. Das Computerprogramm ist ausgelegt, einen von einer Sensoreinheit erkannten Bestrahlungsbereich mit vordefinierten Bestrahlungsbereichen zu vergleichen. Es ist weiter ausgelegt, eine Emission von Lichtstrahlung umfassend Wellenlängen in einem Bereich von zwischen 280 nm und 315 nm durch mindestens ein erstes Leuchtmittel auszulösen. Dies geschieht dann, wenn der erkannte Bestrahlungsbereich nicht einem von einer Bestrahlung ausgeschlossenen Bestrahlungsbereich entspricht.

Das erfindungsgemässe Computerprogrammprodukt kann somit sicherstellen, dass die Bestrahlung stets gesundheitlichen Vorgaben entsprechen kann. Besonders bevorzugt sind die geeigneten Bestrahlungsbereiche vordefinierbar.

In einer besonderen Ausführungsform ist ein von einer Bestrahlung ausgeschlossener Bestrahlungsbereich ein Bestrahlungsbereich, ausgewählt aus der Gruppe bestehend aus: ein menschliches Gesicht, ein mit einem oder mehreren Pigmentnävii versehener Hautbereich, ein mit Schorf-, Wund- und/oder Narbengewebe versehener Hautbereich, und ein innerhalb eines vordefinierten Zeitintervalls bereits mit Lichtstrahlung umfassend Wellenlängen in einem Bereich von zwischen 280 nm und 315 nm bestrahlter Hautbereich.

In einer besonderen Ausführungsform ist ein von einer Bestrahlung ausgeschlossener Bestrahlungsbereich ein Bestrahlungsbereich, der zu einer Person gehört, welche nicht für eine Nutzung der Vorrichtung zugelassen sind. Dies kann zum Beispiel über eine Registrierung sichergestellt werden, und durch ein Hinterlegen eines Gesichtsbildes als biometrisches Erkennungsmittel. Zudem können auf diese Weise Minderjährige als von der Nutzung der Vorrichtung ausgeschlossen werden.

In einer besonderen Ausführungsform ist das Computerprogrammprodukt ausgelegt, um einem bestimmten Bestrahlungsbereich ein Zeitintervall zuzuordnen. Innerhalb dieses Zeitintervalls ist der Bestrahlungsbereich mit Lichtstrahlung umfassend Wellenlängen in einem Bereich von zwischen 280 und 315 nm zu bestrahlen. Dies kann z.B. in Wirkweise mit der genannten Vorrichtung erfolgen, indem die Steuereinheit einen bestimmten Bestrahlungsbereich identifiziert und als geeignet erkannt wird. Die Bestrahlung wird initiiert und ein bestimmter / ein bestimmtes Zeitintervall festgelegt, in dem die Bestrahlung erfolgen soll. Dieser Zeitintervall kann fix ausgelegt sein, z.B. einen Zeitintervall von zwischen einer halben und zehn Minuten, insbesondere zwischen einer und acht Minuten, weiter insbesondere zwischen drei und fünf Minuten, oder er kann variabel sein und anhand von Parametern definiert werden, die z.B. vorgängig mit einem Benutzerprofil hinterlegt worden sind, die Hauttyp, Vitamin-D-Bedarf, Lebensweise etc. umfassen können. So ist z.B. bekannt, dass je dunkler der Hauttyp ist, desto länger ein Aufenthalt in der Sonne erfolgen muss, bis eine ausreichende Menge Vitamin D3 synthetisiert werden konnte. Weiter kann eine Lebensweise, die z.B. stark in den Nachtstunden erfolgt oder jenseits eines Breitengrades stattfindet, bei dem eine tägliche Sonneneinstrahlung ein gewisses Mass unterschreitet, ebenfalls ein Anlass sein, die entsprechenden Zeitintervalle anzupassen. Auch eine medizinische Vorgeschichte kann Anlass sein, um eine entsprechende Anpassung vorzunehmen, so z.B. eine Störung im Vitamin-D-Stoffwechsel oder ein besonderer Bedarf an Vitamin D aufgrund des Alters und/oder medizinischer Gebrechen.

In einer besonderen Ausführungsform ist das Computerprogrammprodukt ausgelegt, eine Suche eines vordefinierten Bestrahlungsbereichs zu steuern, indem das Computerprogrammprodukt die Sensoreinheit steuert. Die Sensoreinheit kann ausgelegt sein, wenn kein geeigneter Bestrahlungsbereich gefunden wird, einen Suchlauf vorzunehmen, indem der gesamte Wirkbereich gescannt wird und ein möglicher Bestrahlungsbereich identifiziert wird.

In einer besonderen Ausführungsform ist das Computerprogrammprodukt ausgelegt, jedes von einem optischen Sensor aufgenommene Bild mit einem von einer Bestrahlung ausgeschlossenen Bestrahlungsbereich zu vergleichen.

So kann sichergestellt werden, dass nicht aus Versehen ein Bestrahlungsbereich beaufschlagt wird, der nicht für die Beaufschlagung vorgesehen ist.

In einer besonderen Ausführungsform ist das Computerprogrammprodukt ausgelegt, durch ein Signal der Sensoreinheit einen Betriebsmodus aufzunehmen, insbesondere einen geeigneten, nicht von einer Bestrahlung ausgeschlossenen Bestrahlungsbereich zu suchen. Dieses Signal kann z.B. eine Bewegungsdetektion sein.

In einer weiteren besonderen Ausführungsform ist das Computerprogrammprodukt ausgelegt, ein Aussetzen einer Bestrahlung eines Bestrahlungsbereichs mit Lichtstrahlung umfassend die Wellenlängen in einem Bereich von zwischen 280 und 315 nm zu veranlassen, sobald eine Sensoreinheit einen von einer Bestrahlung ausgeschlossenen Bestrahlungsbereich im Wirkbereich des Leuchtmittels erkennt. Dies kann z.B. dann erfolgen, wenn eine bestrahlte Person sich bewegt oder eine weitere Person in diesen Bestrahlungsraum tritt, sodass nicht mehr gewährleistet sein kann, dass ein von der Bestrahlung ausgeschlossener Bereich, wie z.B. ein Gesicht, nicht von der Strahlung betroffen wird.

Mit der erfindungsgemässen Vorrichtung und dem entsprechenden Computerprogrammprodukt wird ein Mittel bereitgestellt, um eine in vielen Bereichen einsetzbare Vorrichtung zur Stimulation der Biosynthese von Vitamin D3 bereitzustellen. Die gezeigte Vorrichtung ist sicher in der Anwendung, verhindert viele negative Nebeneffekte einer Bestrahlung mit UV-B-Strahlung, also Strahlung in einem Wellenlängenbereich von zwischen 280 und 315 nm.

Im Folgenden wird die erfindungsgemässe Vorrichtung nun anhand von konkreten Ausführungsbeispielen und Figuren näher erläutert, ohne jedoch an diese gebunden zu sein. Für den geneigten Fachmann ergeben sich allerdings aus diesen konkreten Ausführungsbeispielen weitere vorteilhafte Ausführungsformen der erfindungsgemässen Vorrichtung.

Insbesondere die Figuren illustrieren eine Umsetzung auf schematische Art und Weise, wobei bei den Bezugszeichen das gleiche Element jeweils mit dem gleichen Bezugszeichen zur einfacheren Nachverfolgung versehen ist.

### Figurenbeschrieb

Anhand der nachfolgenden Figuren werden Ausführungsbeispiele der Erfindung beschrieben.

Es zeigen:
- Fig. 1a: eine erfindungsgemässe Vorrichtung zur Stimulation der Biosynthese von Vitamin D3.
- Fig. 1b: die Vorrichtung der Fig. 1a in Frontalansicht;
- Fig. 1c: einen Querschnitt durch die Vorrichtung der Fig. 1b in der Ebene C-C;
- Fig. 1d: eine Seitenansicht der Vorrichtung der Fig. 1b;
- Fig. 1e: einen Seitenquerschnitt der Vorrichtung der Fig. 1b in der Ebene A-A;
- Fig. 1f: einen Seitenquerschnitt der Vorrichtung der Fig. 1b in der Ebene B-B;
- Fig. 1g: eine Explosionsdarstellung der Vorrichtung der Fig. 1a;
- Fig. 1h: eine Detailansicht eines Leuchtmittels der Fig. 1a;
- Fig. 1i: eine alternative Anordnung eines Leuchtmittels für eine Vorrichtung gemäss Fig. 1a;
- Fig. 2: eine alternative Ausführungsform einer erfindungsgemässen Vorrichtung;
- Fig. 3a: eine weitere alternative Ausführungsform einer erfindungsgemässen Vorrichtung in Frontalansicht, und
- Fig. 3b: die erfindungsgemässe Vorrichtung gemäss Fig. 3a in isometrischer Ansicht.

### Ausführung der Erfindung

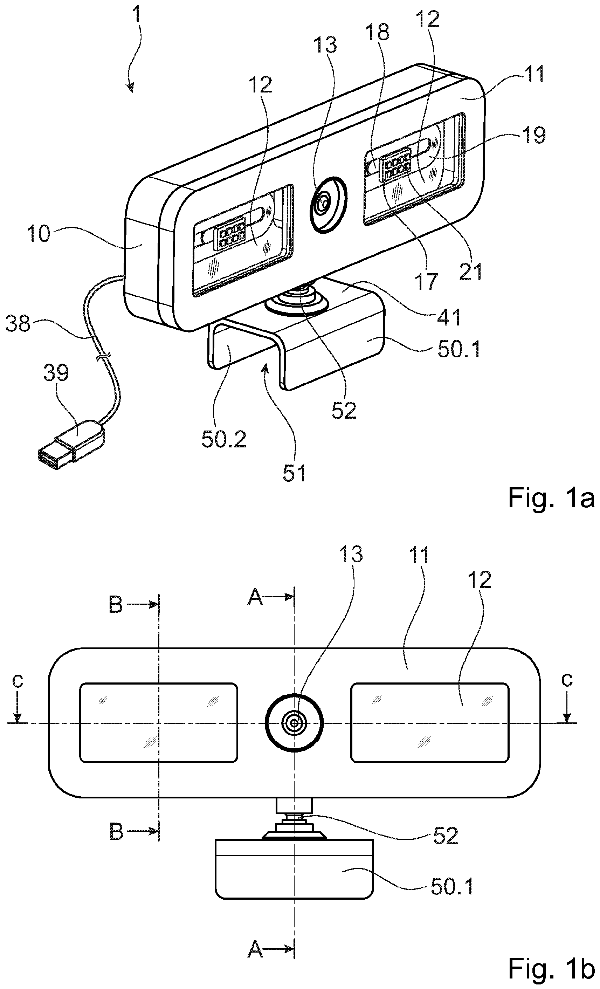

In der **Fig. 1a** ist eine erfindungsgemässe Vorrichtung 1 zur Stimulation der Biosynthese von Vitamin D3 gezeigt. Die Vorrichtung 1 ist geeignet, um z.B. im privaten Bereich verwendet zu werden und dort eine Ergänzung zu einem gesunden und bewussten Lebensstil zu sein. Die Vorrichtung 1 umfasst ein Gehäuse 10. Im vorliegenden Beispiel ist das Gehäuse 10 aus Aluminium in einem Tiefzugverfahren hergestellt. Selbstverständlich sind auch Gehäuse aus Kunststoff oder aus Edelstahl oder Keramik geeignet. Ein besonderer Vorteil von Aluminium ist die gute Wärmeleitfähigkeit, was helfen kann, allfällig entstehende Abwärme an die Umgebung abzuführen. Das Gehäuse 10 ist schalenförmig aufgebaut und auf einer Frontseite von einer Frontplatte 11 begrenzt. Die Frontplatte 11 weist zwei Glasfenster 12 auf, durch die eine Lichtstrahlung vom Inneren des Gehäuses 10 nach aussen dringen kann. Obschon an dieser Stelle von Glasfenster 12 die Rede ist, kann das gezeigte Sichtfenster ebenso aus einem Kunststoff sein, z.B. einem Plexiglas. Idealerweise wird das Glas so ausgewählt, dass es im Wesentlichen durchlässig für UV-B-Strahlung in den Wellenlängen von zwischen 280 nm und 315 nm ist. Ein geeignetes UV-permeables Glas wird z.B. in der US 5474589 A (Sumitomo Electric Industries Ltd.) gezeigt.

Durch das Glasfenster 12 ist das Leuchtmittel erkennbar. Im vorliegenden Beispiel setzt sich das Leuchtmittel zusammen aus einem Array 17, welches eine Mehrzahl an UV-LED 21 aufweist. Der Array 17 ist horizontal verschiebbar gelagert, indem er über einen Leuchtmittelschlitten 18 in einem Leuchtmittelrahmen gelagert ist. Das Gehäuse 10 ist über einen mit einem Lagergelenk mit dem Gehäuse 10 verbundenen Ständer 52 mit einem Standfuss 41 verbunden. Zur Befestigung auf einer Kante verfügt der Standfuss 41 über einen Greifer, umfassend einen ersten Greifer 50.1 und einen zweiten Greifer 50.2, die eine mittig zwischen dem ersten und dem zweiten Greifer 50.1, 50.2 verlaufende Haltenut schliessen. Bevorzugt sind der erste und der zweite Greifer 50.1, 50.2 gummiert, sodass eine zusätzliche Reibung entsteht und den Griff des Greifers verbessert. Die gezeigte Vorrichtung 1 verfügt über einen Stromanschluss über eine Kabelverbindung 38, welche in einen USB-/Stromstecker 39 endet. In der Mitte der Frontplatte 11 befindet sich die Kameralinse 13, hinter der eine Kamera als optischer Sensor verbaut ist.

Die in der Fig. 1a gezeigte Vorrichtung 1 kann z.B. an einer Oberkante eines Computerbildschirms, eines Fernsehers oder einer Stativeinrichtung platziert werden, und eine Grundeinstellung der Ausrichtung der Vorrichtung 1 kann über den Ständer 52 erfolgen. Die Kameralinse 13 kann einen Frontbereich überwachen und darin allfällige Bestrahlungsbereiche erkennen. Wird ein Bestrahlungsbereich von einer Steuereinheit (in der Fig. 1a nicht gezeigt) identifiziert und unterliegt der Bestrahlungsbereich nicht einem Vorbehalt, d.h. handelt es sich beim Bestrahlungsbereich nicht um einen von der Bestrahlung ausgeschlossenen Bestrahlungsbereich, so wird über das Leuchtmittel, resp. im vorliegenden Beispiel durch die UV-LED 21 Lichtstrahlung emittiert. Dazu können die LEDs 21 entsprechende Reflektoren aufweisen, welche die Lichtstrahlung bündeln und entsprechend auf den Bestrahlungsbereich begrenzen (in der Fig. 1a nicht gezeigt).

Im Betrieb kann die Vorrichtung 1 zahlreiche Daten mit einem entsprechenden Computer austauschen. So kann über den USB-/Stromstecker 39 eine Überwachung der Betriebsparameter erfolgen, wie z.B. Betriebsdauer, Temperatur, Bestrahlungsdauer einzelner Bestrahlungsbereiche, sowie wie oft ein bestimmter Bestrahlungsbereich ausgespart werden musste (z.B. das Gesicht).

Die Kamera hinter der Kameralinse 13 weist eine Auflösung auf, die ausreichend ist, um der Steuereinheit das Erkennen eines Gesichts zu ermöglichen. In der einfachsten Ausführung der vorliegenden Erfindung ist die Vorrichtung 1 lediglich dafür ausgebildet, ein Gesicht als solches zu erkennen, um es entsprechend von einer Bestrahlung auszusparen, resp. die Bestrahlung auszusetzen, wenn ein Gesicht im Bestrahlungsbereich ist.

Für die LED ist entscheidend im vorliegenden Beispiel, dass ein Peak des emittierten Wellenlängenspektrums im Bereich von zwischen 280 nm und 315 nm liegt. Im vorliegenden Beispiel wurde eine LED mit einem Peak von 298 nm verwendet. Solche LEDs sind unter anderem von Dowa Electronic Materials Co., Ltd. (Japan) erhältlich.

Mit dieser Ausgestaltung wurde gefunden, dass eine gesamte tägliche Bestrahlungsdauer mit von 13 bis 14 Minuten bei einer Dosis von **12.5 mJ/cm²** mit einer erythemalen Bestrahlungsstärke von 0.015 mW/cm² geeignet sein kann, um bei einem Benutzer vom Hauttyp 2 die körpereigene Biosynthese von Vitamin D3 zu stimulieren und zu ergänzen.

In der **Fig. 1b** ist die Vorrichtung 1 der Fig. 1a in frontaler Ansicht nochmals gezeigt, wobei nur die Frontplatte 11 mit den entsprechenden Glasfenstern 12 und der Kameralinse 13 von vorne ersichtlich sind. Ebenfalls gut erkennbar sind der Ständer 52, sowie der am Standfuss 41 ausgebildete erste Greifer 50.1. Die Fig. 1b dient dazu, die Schnittebenen zu definieren, welche in den Figuren, die folgend im Detail besprochen werden, wobei im Detail ein Längsquerschnitt C-C, sowie zwei Profilquerschnitte A-A und B-B im Detail dargestellt werden.

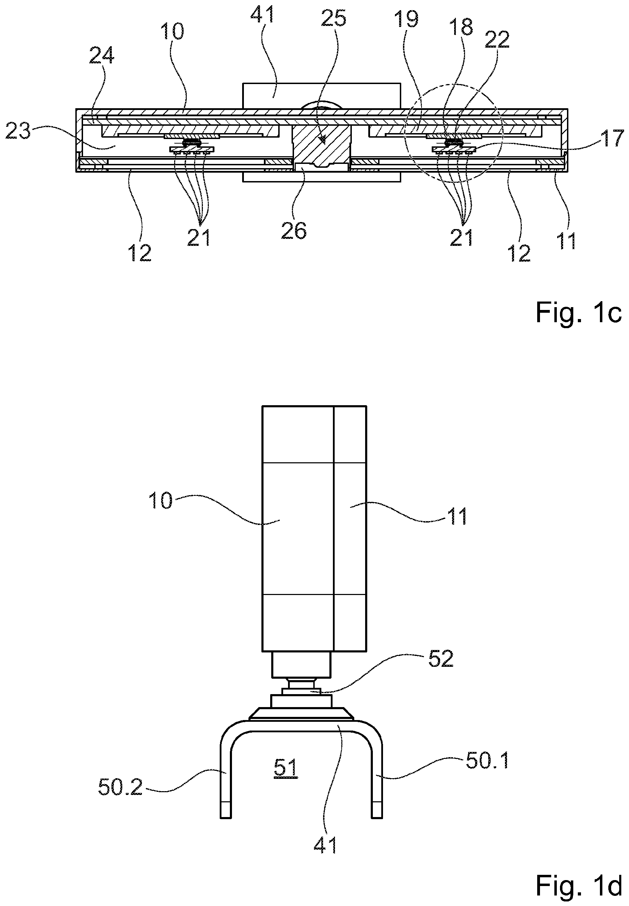

Die **Fig. 1c** zeigt den genannten Längsquerschnitt C-C der Fig. 1b in Aufsicht, d.h. von oben auf die Vorrichtung 1. Der Querschnitt C-C verläuft durch die Mitte der Höhenausdehnung der Vorrichtung 1 und somit durch die Mitte der Kamera und der Linse 13, welche im vorliegenden Fall im Kameraraum des Gehäuses 10 untergebracht sind. Der Kameraraum 25 wird von einer Kameralinse 26, welche bündig mit der Stirnseite der Frontplatte 11 ausgelegt ist, abgeschlossen. Im Kameraraum 25 kann eine gängige Kamera platziert werden, welche geeignet ist, ein optisches Bild der Umgebung zu erfassen. Dabei sollte die Auflösung der Kamera so gewählt sein, dass diese in der Lage ist, von einer Steuereinheit ausgewertet zu werden und eine Gesichtserkennung möglich ist. Die Kameralinse 26 kann so gewählt sein, dass sie einen weiten Winkel erfasst, z.B. indem eine entsprechende Wölbung oder ein Schliff in der Linse den erfassten Bereich vergrössert. Solche Kameras sind vor allem im Überwachungsbereich verbreitet und können vom Fachmann bei Bedarf unter Berücksichtigung der Einbaugrösse ausgewählt werden. Die Kamera bildet die Sensoreinheit der erfindungsgemässen Vorrichtung. Auf beiden Seiten des Kameraraums 25 befindet sich ein Leuchtmittelraum 23, in dem zwei Leuchtmittel untergebracht sind. Die Leuchtmittel umfassen eine Mehrzahl an UV-LEDs und sind beweglich ausgebildet. Insgesamt ist der gesamte Leuchtmittelraum als Bewegungsraum für die LED ausgebildet. Die LEDs sind an einem Array angebracht und über einen Schwenkarm 22, welcher den Array 17 normal zur Papierebene in einem Winkel von zwischen ± 45° verschwenkbar gestalten. Der Schwenkarm 22 ist mit einem Leuchtmittelschlitten 18 verbunden, der in einer entsprechenden Nut des Leuchtmittelrahmens horizontal verschiebbar gelagert ist. Zur Bewegung kann z.B. ein einfaches Zahnwerk mit einem Bandantrieb vorgesehen sein.

Die Kamera (nicht gezeigt) sowie die beiden Leuchtmittel sind an einer Rahmenplatte 24 montiert, welche zusätzlich die elektronischen Zuleitungen umfassen kann (nicht im Detail gezeigt). Zusätzlich kann die Rahmenplatte 24 die Steuerung umfassen. Die Rahmenplatte 24 kann im Wesentlichen oder aus einzelnen Teilen als Wärmetauscher fungieren und allfällig erzeugte Abwärme von den Leuchtmitteln auf das Gehäuse und entsprechend am Gehäuse ausgebildete Wärmetauscherelemente, wie z.B. Lüftungsschlitze und/oder Lamellen, ableiten. Ebenfalls in Aufsicht sichtbar ist der Standfuss 41. Im Betrieb würden die Leuchtmittel jeweils synchron zueinander operiert oder unabhängig voneinander, z.B. um einen bestimmten Bereich zu fokussieren. Grundsätzlich umfassen die Leuchtmittel zusätzlich Reflektoren oder die Glasfenster 12 sind mit entsprechenden Linsen ausgestaltet, um die Lichtstrahlung durch die Leuchtmittel im Wesentlichen zu sammeln, sodass möglichst definierte Bestrahlungsbereiche entstehen. Die Vorrichtung kann ausgebildet sein, anhand des Kamerabildes festzustellen, ob eine Person in einer geeigneten Distanz zur Vorrichtung steht, um die Bestrahlung zu entscheiden.

Die beispielhafte Vorrichtung ist vorzugsweise in einem Badezimmerbereich platzierbar, wo z.B. eher freie Hautbereiche als Bestrahlungsbereiche bestehen. Eine geeignete Montageanordnung ist z.B. oberhalb des Badezimmerspiegels. Durch eine zuverlässige Gesichtserkennung wird verhindert, dass trotz dieser Lage keine schädliche UV-B-Strahlung auf die Augen gerichtet wird.

Die **Fig. 1d** zeigt die Vorrichtung in Seitenansicht, wobei das Gehäuse 10 von der Frontplatte 11 stirnseitig abgeschlossen wird und die so resultierende Gehäusekombination über einen Ständer 52 mit einem Standfuss 41 verbunden wird. Die beiden Greifer 50.1, 50.2 umschliessen eine Haltenut 51.

In einer besonderen Ausführungsform sind die Greifer 50.1, 50.2 aus einem flexiblen, elastischen Material, sodass sie eine Rückstellkraft ausüben auf einen Gegenstand in der Haltenut 51.

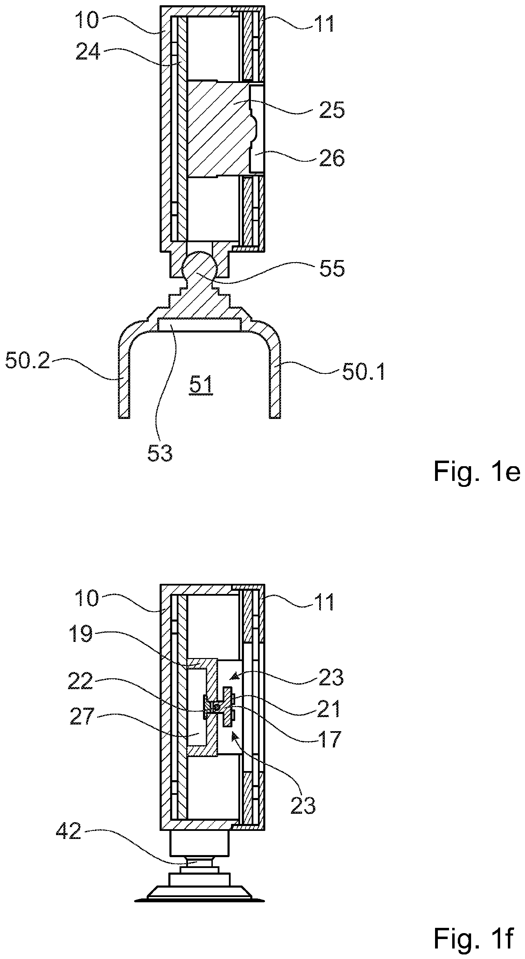

Die **Fig. 1e** zeigt den Querschnitt durch die Mitte der Vorrichtung in der Ebene A-A. Zusätzlich oder als Alternative zu der in der Fig. 1d gezeigten Halterung über flexible Greifer 50.1, 50.2 verfügt diese Ausführungsform über einen Magneten 53, welcher zusätzlich eine Sicherung der Vorrichtung 1 an einem metallischen und/oder magnetischen Halteelement innerhalb der Haltenut 51 ermöglicht. Die gesamte Halterung ist über ein Kugelgelenk 55 mit dem Gehäuse 10 verbunden. Im Gehäuse 10 ist eine Rahmenplatte 24 vorgesehen, welche in der Mitte einen Kammerbereich für einen Kameraraum 25 trägt, der stirnseitig von einer Kameralinse 26 abgeschlossen wird. Die Stirnseite wird zudem von der Frontplatte 11 abgeschlossen.

In der **Fig. 1f** **ist** ein entsprechender Querschnitt durch die Achse B-B gezeigt, in welchem die Leuchtmittel untergebracht sind. Der Ständer 42 wird hier in Seitenansicht gezeigt. Die Leuchtmittel befinden sich in einem Leuchtmittelraum 23. Bei Bedarf können kleine Miniventilatoren vorgesehen sein, um den Leuchtmittelraum 23 mit einem Luftstrom zu beaufschlagen. Diese Miniventilatoren können über entsprechende Lüftungsschlitze im Gehäuse 10 die Abwärme vom Leuchtmittelraum 23 nach draussen / aussen tragen und z.B. bei intensivem Betrieb des Leuchtmittels dieses kühlen (nicht gezeigt). Auf der Rahmenplatte angebracht, ist ein Leuchtmittelrahmen 19, der eine Schiene aufweist, in welchem ein Array 17 mit mehreren UV-LED 21 normal zur Schnittebene verschiebbare Leuchtmittel angebracht sind. Hinter dem Leuchtmittelrahmen 19, zwischen Leuchtmittelrahmen 19 und Rahmenplatte 24, befindet sich ein Steuerungsraum 27. Im Steuerungsraum sind die Kabelführungen für die Stromzufuhr des Leuchtmittelschlittens, des Schwenkarms 22, der Leuchtmittel und allfälliger zusätzlicher Elektronik untergebracht.

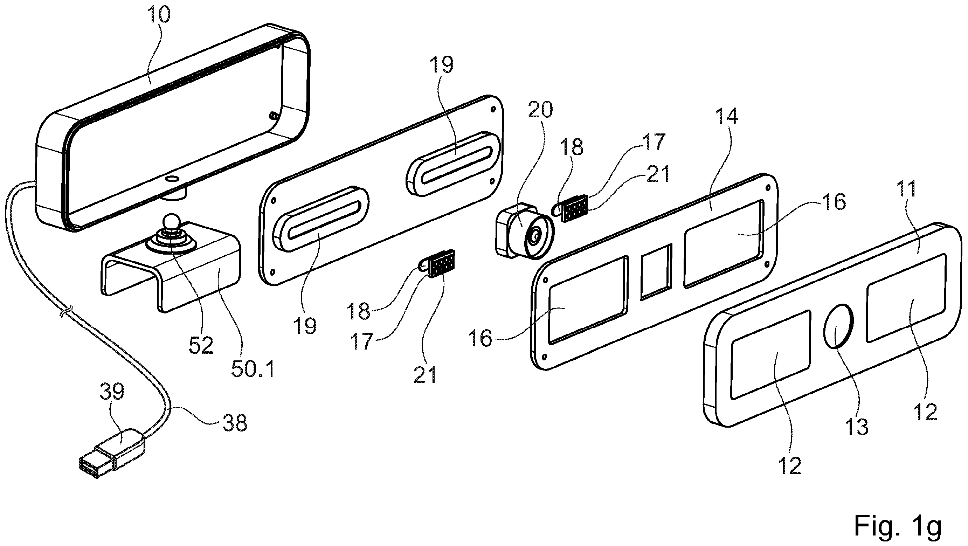

In der **Fig. 1g** ist die Vorrichtung der Fig. 1a in Explosionsdarstellung gezeigt, um die einzelnen Elemente nochmals klar zu definieren. Eine Frontplatte 11 schliesst das Gerät stirnseitig ab und weist mehrere Ausnehmungen 12, 13 auf. Eine zentrale Ausnehmung 13 dient dazu, eine Kameralinse aufzunehmen, resp. ermöglicht es einer Kamera 20, ein Bild von der Umgebung vor der Vorrichtung 1 aufzunehmen. Auf beiden Seiten der Kameralinse 13 sind zwei Glasfenster 12 vorgesehen, die UV-B-strahlungspermeabel sind. Eine Trägerplatte 14 definiert durch zwei Trägerplattenausnehmungen 16 einen Leuchtmittelraum 23 und befindet sich direkt hinter den Glasfenstern 12 in einer Front zur Rückseitenbetrachtung. Zwei Leuchtmittel, umfassend LED 21, Array 17 und Leuchtmittelschlitten 18, sind beidseitig einer Kamera 20 angeordnet und werden in einem Leuchtmittelrahmen 19 untergebracht. Der Leuchtmittelrahmen 19 ist an einer Rahmenplatte 24 befestigt. Die Bauteile werden in einem Gehäuse 10 untergebracht, welches über ein Kugelgelenk mit einem Standfuss verbunden ist, welches über zwei Greifer 50.1, 50.2 verbunden ist. Aus dem Gehäuse 10 wird ein Kommunikationselement, resp. eine Stromzufuhr aus dem Geräteinnen nach aussen geführt über eine Kabelverbindung 38 mit einem USB-/Stromstecker 39.

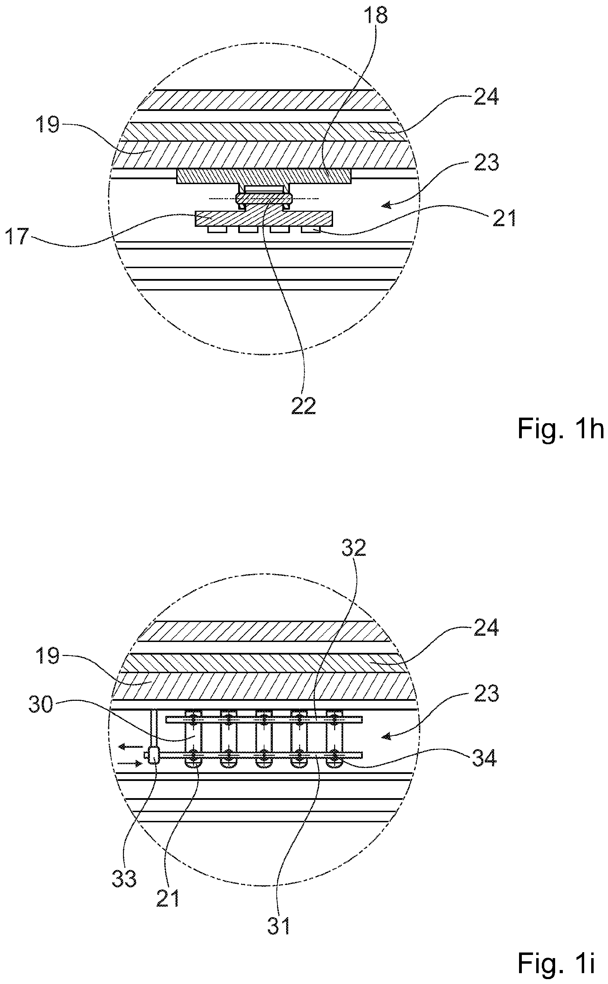

Die **Fig. 1h** zeigt im Detail die Anordnung eines Leuchtmittels in einem Bestrahlungsraum 23. Das Leuchtmittel umfasst eine Mehrzahl an LED 21, welche ausgelegt sind, UV-B-Strahlung zu emittieren. Bevorzugt weisen die genannten UVBs einen Peak in einem Bereich von zwischen 290 und 298 nm auf. Die LEDs sind an einem Array 17 angebracht, welcher über einen Schwenkarm 22 einen Winkel von zwischen ± 45° abschwenken kann. Der Schwenkarm 22 ist an einem Leuchtmittelschlitten montiert, welcher wiederum in einem Leuchtmittelrahmen 19 bewegbar gelagert ist. Die Elektronik der erfindungsgemässen Vorrichtung ist ausgelegt, um den Schwenkarm und den Leuchtmittelschlitten unabhängig zu bewegen, sodass ein breites Spektrum an Bestrahlungsbereichen abgedeckt werden kann.

In der **Fig. 1h** nicht gezeigt sind einzelne Reflektoren, wobei jedes LED einen Reflektor aufweist, sodass die Gesamtstrahlung aus dem Leuchtmittel gezielt einen bestimmten Bestrahlungsbereich erfassen kann und definiert / erfassen und definieren kann.

Eine Alternative zu der in der Fig. 1h gezeigten Bauweise ist in der **Fig. 1i** ersichtlich. Auch in der Fig. 1i erstrecken sich die Leuchtmittel in den Leuchtmittelraum 23. Im Unterschied zu der Fig. 1h sind die einzelnen LED 21 mit einem Leuchtmittelkörper verbunden, welcher auf zwei Achsen mittels einer Antriebsleiste 31 und einer hinteren Leiste 32 verschwenkbar sind. Ein Antriebsleistenmotor 33 bewegt die Antriebsleiste in der in Pfeilrichtung gezeigten Richtung hin und zurück, während die hintere Leiste entsprechend ebenfalls bewegt wird. Dadurch lassen sich die Leuchtmittelkörper ausrichten. Wird eine solche Anordnung ebenfalls in der horizontalen Richtung vorgesehen mit einer entsprechenden Antriebsleiste und einem entsprechenden Antriebsleistenmotor, so lassen sich die LEDs somit dergestalt verschwenken, dass ein breiter Kegel davon erfasst von einem Lichtstrahl erfasst werden kann.

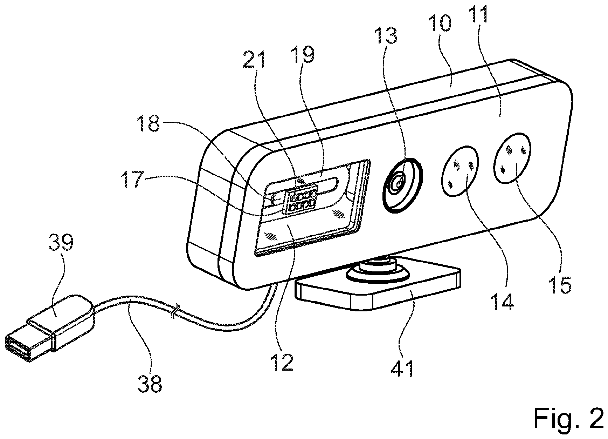

In der **Fig. 2** wird eine alternative Ausführungsform der Vorrichtung gezeigt. In wesentlichen Teilen umfasst die Vorrichtung der Fig. 2 den gleichen Aufbau, wie der in der Vorrichtung 1, allerdings umfasst die Vorrichtung der Fig. 2 zusätzliche Leuchtmittel, nämlich ein zusätzliches zweites Leuchtmittel und ein zusätzliches drittes Leuchtmittel. Dazu bestehen Glasscheiben: eine erste Glasscheibe für das zweite Leuchtmittel (14) und eine zweite Glasscheibe für das dritte Leuchtmittel (15). Diese sind als Ausnehmung in einer Frontplatte vorgesehen, welche wie in der Fig. 1 zusätzlich eine Kameralinse 13, sowie ein Glasfenster 12 mit dahinterliegendem Leuchtmittel analog, wie es in der Fig. 1 beschrieben wurde. Dieses Leuchtmittel ist eine UV-LED-Diode 21 auf einem Array 17, welche bewegbar angeordnet ist, nämlich über eine Schiene, welche von einem Leuchtmittelschlitten 18 auf einem Leuchtmittelrahmen 19 abgefahren werden kann. Die Vorrichtung kann mittels eines USB-/Stromsteckers 39 mit einem Stromanschluss, resp. einem Computer verbunden werden und so den Datenaustausch ermöglichen. Wie bereits im allgemeinen Teil beschrieben, kann eine erfindungsgemässe Vorrichtung selbstverständlich auch ein Modul zur kabellosen Kommunikation umfassen, wie z.B. ein WiFi- oder Bluetooth-Modul umfassen. Je nach Ausgestaltung kann mehr oder weniger die Steuerung direkt in der erfindungsgemässen Vorrichtung stattfinden. So kann die Vorrichtung einen Prozessor umfassen, welcher die Steuerungsaufgaben, wie sie im allgemeinen Teil beschrieben sind, und das Ausführen des Computerprogrammproduktes übernimmt. Ebenso ist denkbar, dass ein Drittcomputer, z.B. ein über den USB-Anschluss verbundener Computer, diese Arbeiten übernimmt.

Zurück zum Beispiel der Fig. 2, wo zusätzlich zum UV-B-Leuchtmittel ein zweites Leuchtmittel mit sichtbarem Licht vorgesehen ist, und ein drittes Leuchtmittel mit Licht im nahen Infrarotbereich, welches nicht sichtbar ist, aber die Durchblutung des bestrahlten Gewebes verbessern kann. Das Licht im sichtbaren Spektrum kann neben einem positiven psychosomatischen Effekt, z.B. indem Licht in einer angenehmen Lichtfarbe ausgestrahlt wird, zusätzlich den Betrieb der Vorrichtung anzeigen. Vorzugsweise handelt es sich bei diesem ersten und bei diesem zweiten Leuchtmittel ebenfalls um LED-Leuchtmittel, welche einen Peak oder ein Spektrum in den Wellenlängenbereichen 380 nm bis 720 nm für das zweite Leuchtmittel, resp. 720 nm und 1.800 nm für das dritte Leuchtmittel aufweisen.

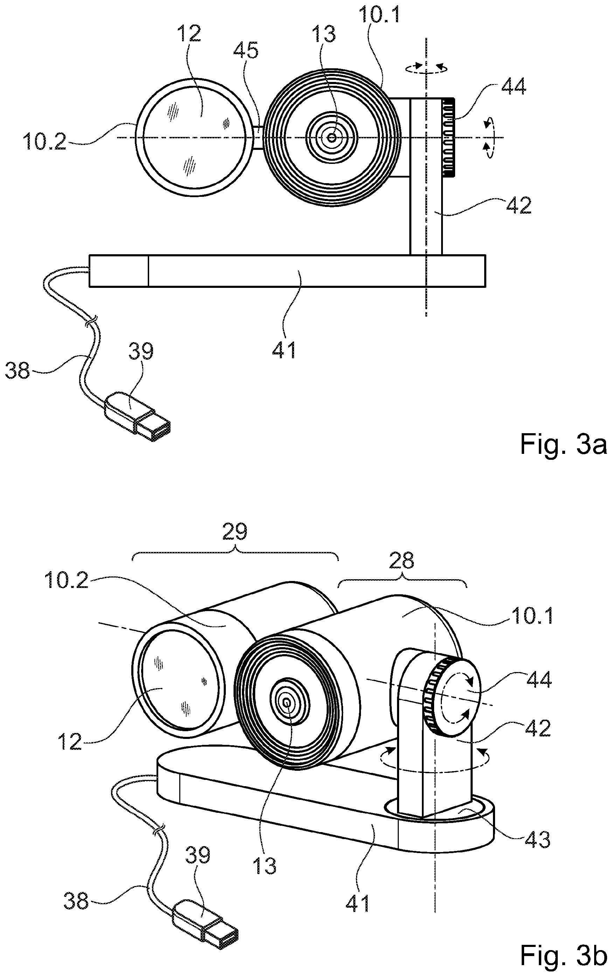

Eine weitere alternative Ausführungsform ist in der **Fig. 3a** dargestellt. In dieser Ausführungsform der vorliegenden Erfindung ist das Leuchtmittel mit der Sensoreinheit physikalisch gekoppelt, sodass eine Richteinheit beide synchron bewegt. Mit anderen Worten ist das Leuchtmittel immer auf den Kamerabereich ausgerichtet. Dazu weist die erfindungsgemässe Vorrichtung ein zweiteiliges Gehäuse 10.1, 10.2 auf. Das erste Teil des zweiteiligen Gehäuses 10.1 beherbergt die Sensoreinheit, im vorliegenden Beispiel eine Kamera mit einer Kameralinse 13. Das zweite Teil des zweiteiligen Gehäuses 10.2 indessen beherbergt das Leuchtmittel hinter einem Glasfenster 12. Eine Verbindung 45 zwischen dem ersten Teil des zweiteiligen Gehäuses 10.1 und dem zweiten Teil des zweiteiligen Gehäuses 10.2 ist starr und ermöglicht es, mittels einer Richteinheit beide Elemente gleichzeitig zu bewegen. Die Richteinheit umfasst in dieser Ausführungsform einen rotierbar gelagerten Werkzeugarm 44 und einen rotierbar gelagerten Ständerboden 43. Dazwischen erstreckt sich ein Ständer. Dadurch lässt sich die erfindungsgemässe Vorrichtung in einem Halbkugelbereich um einen Standfuss 41 ausrichten, sodass insgesamt ein sehr breiter Wirkbereich von einem Leuchtmittelelement 29 und einem Kameraelement 28 erfasst werden kann.

Auch diese Ausführungsform bezieht ihre Energie über einen USB-/Stromstecker 39.

Die erfindungsgemässe Vorrichtung ist mit einer Steuereinheit ausgestattet. In allen genannten Beispielen ist die Steuereinheit so ausgelegt, dass sie den Bestrahlungsbereich zu identifizieren vermag. Die entsprechenden Sensoreinheiten, also in den gezeigten Beispielen die Kameraelemente, erkennen einen Bestrahlungsbereich und liefern ein Bild an die Steuereinheit. Diese ist ausgelegt, einen Bestrahlungsbereich zu identifizieren, d.h. den erkannten Bestrahlungsbereich auszuwerten und festzustellen, ob es sich bei diesem Bestrahlungsbereich allenfalls um einen von der Bestrahlung ausgeschlossenen Bestrahlungsbereich handelt. Ob ein Bestrahlungsbereich von der Bestrahlung ausgeschlossen ist, kann anhand einer vordefinierten Datenbank, welche z.B. in einer Speichereinheit (in den Figuren nicht gezeigt) hinterlegt ist. Es ist ebenfalls denkbar, dass das Computerprogrammprodukt, welches die Steuerung übernimmt, in der Lage ist, mittels einer intelligenten Programmierung anhand vordefinierter Parameter zu bestimmen, ob es sich beim Bestrahlungsbereich um einen solchen ausgeschlossenen Bestrahlungsbereich handelt. Dazu können bestimmte Parameter vorgesehen sein, wie z.B. Form und Anordnung von erkennbaren Mustern im erfassten Bildbereich und Zuordnung dieser erkennbaren Muster an bestimmte Elemente in ein für einen Bestrahlungsbereich, welcher von der Bestrahlung ausgeschlossen ist. Typischerweise handelt es sich dabei um Elemente, die für die Gesichtserkennung eingesetzt werden, wie z.B. Position und Abstand von Augen, Nasen und Mund zur Erkennung eines menschlichen Gesichts. Es ist ebenfalls denkbar, dass die gezeigte Vorrichtung eine Verbindung aufnehmen kann mit einem maschinenlernengestützten Programm. Das Computerprogrammprodukt würde die erkannten Bestrahlungsbereiche mit einer Datenbank vergleichen, resp. anhand von maschinellem Lernen ermitteln, ob es sich bei diesen Bestrahlungsbereichen um allenfalls ausgeschlossene Bestrahlungsbereiche handelt. Zusätzlich ist das Computerprogrammprodukt in der Lage, die Bestrahlung insgesamt zu steuern. Dies kann z.B. dadurch erfolgen, dass das Computerprogrammprodukt ausgelegt ist, die Dauer einer Bestrahlung einem bestimmten Bestrahlungsbereich zuzuordnen. So kann sichergestellt werden, dass der Bestrahlungsbereich nicht zweimal mit der gleichen Strahlen intensität beaufschlagt wird.

Es versteht sich von selbst, dass die erfindungsgemässe Vorrichtung dazu eine Steuereinheit benötigt, welche ausgelegt ist, das genannte Computerprogrammprodukt zu betreiben, und dieses die entsprechenden Anweisungen auszuführen vermag. Dazu kann die Steuereinheit einen entsprechenden Prozessor vorsehen, sowie wahlweise weitere Elemente, wie z.B. einen Rechenspeicher, einen Arbeitsspeicher und/oder eine Ausgabeeinheit. Bei der Ausgabeeinheit kann es sich z.B. um eine einfache Displayanzeige handeln. Die Displayanzeige kann ein initiales Aufsetzen der erfindungsgemässen Vorrichtung erleichtern. Diese Elemente können ganz oder teilweise an ein Drittgerät ausgelagert sein, d.h. sie müssen sich nicht zwangsläufig innerhalb des Gehäuses der oben beschriebenen Vorrichtung befinden. Das Computerprogrammprodukt kann ebenfalls ausgelegt sein, um eine Mehrzahl an erfindungsgemässen Vorrichtungen miteinander zu synchronisieren. So kann das Computerprogrammprodukt z.B. ausgelegt sein, um eine Mehrzahl in einem bestimmten Bereich, wie z.B. einem Gebäude, angeordneten Vorrichtungen miteinander zu vernetzen. Dies kann so weit gehen, dass durch eine Gesichtserkennung, welche das Gesicht einer bestimmten Person zuzuordnen vermag, sichergestellt werden kann, dass die Person innerhalb eines Gebäudes mit der entsprechenden Dosis an Lichtstrahlung beaufschlagt wird und dabei dennoch die entsprechenden Sicherheitsmassnahmen, wie z.B., dass eine bestimmte Hautpartie nie zweimal als Bestrahlungsbereich dienen kann, sichergestellt werden kann.

Idealerweise kann eine Vorrichtung, wie sie in dieser Erfindung gezeigt wird, im privaten Bereich genutzt werden. Besonders geeignet ist die Vorrichtung allerdings in einem Bereich, in dem z.B. die Hautexposition stärker ist als in einem öffentlichen Raum, wo tendenziell mehr Kleidung getragen wird. So sind z.B. Badeanstalten, Schwimmbäder, Wellnessbereiche, das private Badezimmer oder Garderoben ideale Bereiche, in denen eine erfindungsgemässe Vorrichtung, wie hier beschrieben, platziert werden kann. Durch die Lehre der vorliegenden Erfindung ist es möglich, die Stimulation der Biosynthese von Vitamin D3 zu einem beiläufigen Prozess zu machen, was eine gute Compliance durch die Anwender ermöglicht. Somit besteht kein Bedarf mehr, Vitamin-D3-Präparate einzunehmen, welche mitunter vergessen werden können, noch bestimmte rigorose Aussenaufenthalte vorzusehen. Geeignete Anwendungen sind neben den notorischen Breitengraden z.B. Bereiche, in denen aufgrund der Gegebenheit wenig natürliches Sonnenlicht tagsüber hineinscheint, z.B. in unterirdischen Anlagen, grossen Lager- und Fabrikkomplexen, Grossraumbüros, Offshoreanlagen, sowie das Innere von Gebäuden in engen und wenig beschienenen Seiten- und Bergtälern.

### Bezugszeichenliste

- 1): Vorrichtung zur Stimulation des Biosynthese von Vitamin D₃
- 10): Gehäuse
- 10.1): erstes Teil zweiteiliges Gehäuse
- 10.2): zweites Teil zweiteiliges Gehäuse
- 11): Frontplatte
- 12): Glasfenster
- 13): Kameralinse
- 14): Glasscheibe zweites Leuchtmittel
- 15): Glasscheibe drittes Leuchtmittel
- 16): Trägerplattenausnehmungen
- 17): Array
- 18): Leuchtmittel-Schlitten
- 19): Leuchtmittel-Rahmen
- 20): Kamera
- 21): UV-LED-Diode
- 22): Schwenkarm
- 23): Leuchtmittelraum
- 24): Rahmenplatte (Wärmetauscher)
- 25): Kameraraum
- 26): Kameralinse
- 27): Steuerungsraum
- 28): Kameraelement
- 29): Leuchtmittelelement
- 30): Leuchtmittelkörper
- 31): Antriebsleiste
- 32): hintere Leiste
- 33): Antriebsleistenmotor
- 34): Schwenkgelenk
- 38): Kabelverbindung
- 39): USB-/Stromstecker
- 41): Standfuss
- 42): Ständer
- 43): rotierbar gelagerter Ständerboden
- 44): rotierbar gelagerter Werkzeugarm
- 45): Verbindung
- 50.1): erster Greifer
- 50.2): zweiter Greifer
- 51): Haltenut
- 52): Ständer
- 53): Magnet
- 55): Kugelgelenk

## Patentansprüche

1. **Vorrichtung** (1) zur Stimulation der Biosynthese von Vitamin D₃, umfassend
a. Mindestens ein erstes **Leuchtmittel** (21) zur Emission von Lichtstrahlung umfassend Wellenlängen in einem Bereich von zwischen 280 und 315 nm;
c. Eine **Richteinheit** zur Ausrichtung des mindestens einen Leuchtmittels auf den Bestrahlungsbereich, und
d. Eine **Steuereinheit,** welche ausgelegt ist, eine Emission von Lichtstrahlung umfassend Wellenlängen in einem Bereich von zwischen 280 und 315 nm durch das mindestens eine Leuchtmittel auszulösen,
**gekennzeichnet durch**
b. Eine Sensoreinheit zur Erkennung eines Bestrahlungsbereichs;
wobei die Steuereinheit weiters ausgelegt ist, den Bestrahlungsbereich zu identifizieren.

2. Vorrichtung gemäss Anspruch 1, wobei die Sensoreinheit einen **optischen Sensor,** insbesondere eine **Kamera** umfasst.

3. Vorrichtung gemäss einem der Ansprüche 1 oder 2, wobei die Steuereinheit ausgelegt ist, eine Ausrichtung des ersten Leuchtmittels durch die Richteinheit anhand eines identifizierten Bestrahlungsbereichs zu steuern.

4. Vorrichtung gemäss einem der Ansprüche 1 bis 3, umfassend eine **Sammeleinheit** zur Erzeugung eines gerichteten Strahlenbündels aus der Emission von Lichtstrahlung durch das erste Leuchtmittel.

5. Vorrichtung gemäss Anspruch 4, wobei die Sammeleinheit eine **Linse** und/oder einen **Kollimator** umfasst, insbesondere einen Kollimator mit einer **Sammellinse** zur Erzeugung eines gerichteten Strahlenbündels aus der Emission von Lichtstrahlung durch das erste Leuchtmittel.

6. Vorrichtung gemäss einem der Ansprüche 1 bis 5, umfassend ein **zweites Leuchtmittel** zur Emission von Lichtstrahlung umfassend Wellenlängen im sichtbaren Bereich, insbesondere umfassend Wellenlängen in einem Bereich von zwischen 380 nm und 720 nm.

7. Vorrichtung gemäss einem der Ansprüche 1 bis 6, umfassend ein **drittes Leuchtmittel** zur Emission von Lichtstrahlung umfassend Wellenlängen im nahen Infrarotbereich, insbesondere in einem Bereich von zwischen 720 nm und 1800 nm.

8. Vorrichtung gemäss einem der Ansprüche 1 bis 7, wobei die Steuereinheit ausgelegt ist, ein menschliches Gesicht in einem Bestrahlungsbereich zu identifizieren.

9. Vorrichtung gemäss einem der Ansprüche 1 bis 8, wobei die Steuereinheit ausgelegt ist, einen vordefinierten Bestrahlungsbereich zu identifizieren.

10. Vorrichtung gemäss einem der Ansprüche 1 bis 9, umfassend eine **Kommunikationseinheit** zum Austausch von Daten.

11. Vorrichtung gemäss einem der Ansprüche 1 bis 10, wobei das erste Leuchtmittel und/oder das zweite Leuchtmittel und/oder das dritte Leuchtmittel und die Sensoreinheit physisch gekoppelt sind, so dass sie durch die Richteinheit gemeinsam auf den Bestrahlungsbereich richtbar sind.

12. Vorrichtung gemäss einem der Ansprüche 1-11, wobei die Richteinheit ausgelegt ist, in mindestens zwei Achsen verschwenkbar zu sein, insbesondere durch mindestens **einen rotierbar gelagerten Ständerboden** und einen **rotierbar gelagerten Werkzeugarm** zum Verschwenken eines Leuchtmittels auf mindestens zwei Achsen.

13. Vorrichtung gemäss einem der Ansprüche 1 bis 12, wobei der Bestrahlungsbereich ein **Hautbereich** mit einer Fläche von zwischen 40 cm² und 900 cm², insbesondere von zwischen 50 cm² und 600 cm², insbesondere von ungefähr 400 cm², ist.

14. Vorrichtung gemäss Anspruch 13, wobei der Hautbereich durch eine regulierbare **Sammeleinheit** definierbar ist.

15. **Computer Programm Produkt** zur Durchführung einer Steuerung einer Vorrichtung mit einer Sensoreinheit und einem ersten Leuchtmittel zur Stimulation der Biosynthese von Vitamin D₃, insbesondere einer Vorrichtung gemäss einem der Ansprüche 1 bis 13, wobei
a. das Computer Programm Produkt ausgelegt ist, einen von besagter Sensoreinheit erkannten Bestrahlungsbereich mit vordefinierten Bestrahlungsbereichen zu **vergleichen,** und
b. das Computer Programm Produkt ausgelegt ist, eine Emission von Lichtstrahlung umfassend Wellenlängen in einem Bereich von zwischen 280 nm und 315 nm durch mindestens besagtes Leuchtmittel **auszulösen**, wenn der erkannte Bestrahlungsbereich nicht einem von einer Bestrahlung ausgeschlossenen Bestrahlungsbereich entspricht.

16. Computer Programm Produkt gemäss Anspruch 15, wobei ein von einer Bestrahlung ausgeschlossener Bestrahlungsbereich ein Bestrahlungsbereich ist, ausgewählt aus der Gruppe bestehend aus: ein menschliches Gesicht, ein mit einem oder mehreren Pigmentnävi versehener Hautbereich, ein mit Schorf-, Wund- und/oder Narbengewebe versehener Hautbereich, und ein innerhalb eines vordefinierten Zeitintervalls bereits mit Lichtstrahlung umfassend Wellenlängen in einem Bereich von zwischen 280 nm und 315 nm bestrahlter Hautbereich.

17. Computer Programm Produkt gemäss einem der Ansprüche 15 oder 16, wobei das Computer Programm Produkt ausgelegt ist, einem bestimmten Bestrahlungsbereich ein Zeitintervall zuzuordnen, in dem der Bestrahlungsbereich mit Lichtstrahlung umfassend Wellenlängen in einem Bereich von zwischen 280 nm und 315 nm bestrahlt werden soll.

18. Computer Programm Produkt gemäss einem der Ansprüche 15 bis 17, wobei das Computer Programm Produkt ausgelegt ist, eine Suche eines vordefinierten Bestrahlungsbereichs zu steuern, indem das Computer Programm Produkt die Sensoreinheit steuert.

19. Computer Programm Produkt gemäss einem der Ansprüche 15 bis 18, wobei das Computer Programm Produkt ausgelegt ist, jedes von einem optischen Sensor aufgenommene Bild mit einem von einer Bestrahlung ausgeschlossenen Bestrahlungsbereich zu vergleichen.

20. Computer Programm Produkt gemäss einem der Ansprüche 15 bis 19, wobei das Computer Programm Produkt ausgelegt ist, durch ein Signal der Sensoreinheit einen Betriebsmodus aufzunehmen, insbesondere einen geeigneten, nicht von einer Bestrahlung ausgeschlossenen Bestrahlungsbereich zu suchen.

21. Computer Programm Produkt gemäss einem der Ansprüche 15 bis 20, wobei das Computer Programm Produkt ein Aussetzen einer Bestrahlung eines Bestrahlungsbereichs mit Lichtstrahlung umfassend Wellenlängen in einem Bereich von zwischen 280 nm und 315 nm veranlasst, sobald eine Sensoreinheit einen von einer Bestrahlung ausgeschlossenen Bestrahlungsbereich im Wirkbereich des Leuchtmittels erkennt.

22. Computer Programm Produkt gemäss einem der Ansprüche 15 bis 21, weiter ausgelegt, über eine Kommunikationseinheit eine Durchführung einer Steuerung einer Vorrichtung zur Stimulation der Biosynthese von Vitamin D₃ auf einem Drittgerät zu überwachen oder zu konfigurieren.

23. Computer Programm Produkt welches auf einem Drittgerät ausführbar ist, und ausgelegt ist, um Nutzerdaten für eine Vorrichtung gemäss einem der Ansprüche 1 bis 14 zu verwalten,
a. wobei die Nutzerdaten Daten umfassen ausgewählt aus der Gruppe bestehend aus: Hauttyp, Alter, Beruf und Lebenswandel, und
b. wobei das Computer Programm Produkt ausgelegt ist, mit einer Kommunikationseinheit der Vorrichtung besagte Nutzerdaten verschlüsselt auszutauschen.

## Claims

1. A **device** (1) for stimulating the biosynthesis of vitamin D₃, comprising:
a. at least one first **lighting means** (21) for emitting light radiation having wavelengths in a range of between 280 and 315 nm;
c. a **directing unit** for aligning the at least one lighting means with the irradiation region, and
d. a **control unit,** which is configured to trigger emission of light radiation having wavelengths in a range of between 280 and 315 nm by the at least one lighting means,
**characterized by**
b. a sensor unit for detecting an irradiation region;
wherein the control unit is further configured to identify the irradiation region.

2. The device according to claim 1, wherein the sensor unit comprises an **optical sensor,** in particular a camera.

3. The device according to any of claims 1 or 2, wherein the control unit is configured to control an alignment of the first lighting means by means of the directing unit based on an identified irradiation region.

4. The device according to any of claims 1 to 3, comprising a **collecting unit** for generating a directed beam of rays from the emission of light radiation by the first lighting means.

5. The device according to claim 4, wherein the collecting unit comprises a **lens** and/or a **collimator,** in particular a collimator comprising a **collecting lens** for generating a directed bundle of rays from the emission of light radiation by the first lighting means.

6. The device according to any of claims 1 to 5, comprising a **second lighting means** for emitting light radiation having wavelengths in the visible range, in particular having wavelengths in a range of between 380 nm and 720 nm.

7. The device according to any of claims 1 to 6, comprising a **third lighting means** for emitting light radiation having wavelengths in the near infrared range, in particular in a range of between 720 nm and 1800 nm.

8. The device according to any of claims 1 to 7, wherein the control unit is configured to identify a human face in an irradiation region.

9. The device according to any of claims 1 to 8, wherein the control unit is configured to identify a predefined irradiation region.

10. The device according to any of claims 1 to 9, comprising a **communication unit** for exchanging data.

11. The device according to any of claims 1 to 10, wherein the first lighting means and/or the second lighting means and/or the third lighting means and the sensor unit are physically coupled such that they can be jointly directed at the irradiation region by the directing unit.

12. The device according to any of claims 1-11, wherein the directing unit is configured to be pivotable in at least two axes, in particular by at least a **rotatably mounted stand base** and a **rotatably mounted tool arm** for pivoting a lighting means on at least two axes.

13. The device according to any of claims 1 to 12, wherein the irradiation region is a **skin region** having an area of between 40 cm² and 900 cm², in particular of between 50 cm² and 600 cm², in particular of approximately 400 cm².

14. The device according to claim 13, wherein the skin region is definable by an adjustable **collecting unit.**

15. A **computer program product** for executing control of a device comprising a sensor unit and a first lighting means for stimulating the biosynthesis of vitamin D₃, in particular a device according to any of claims 1 to 13, wherein
a. the computer program product is configured to **compare** an irradiation region detected by said sensor unit with predefined irradiation regions, and
b. the computer program product is configured to **trigger** emission of light radiation having wavelengths in a range of between 280 nm and 315 nm by at least said lighting means if the detected irradiation region does not correspond to an irradiation region excluded from irradiation.

16. The computer program product according to claim 15, wherein an irradiation region excluded from irradiation is an irradiation region selected from the group consisting of: a human face, a skin region provided with one or more pigmented nevi, a skin region provided with scab, wound, and/or scar tissue, and a skin region already irradiated within a predefined time interval with light radiation having wavelengths in a range of between 280 nm and 315 nm.

17. The computer program product according to any of claims 15 or 16, wherein the computer program product is configured to assign a time interval to a specific irradiation region in which the irradiation region is to be irradiated with light radiation having wavelengths in a range of between 280 nm and 315 nm.

18. The computer program product according to any of claims 15 to 17, wherein the computer program product is configured to control a search of a predefined irradiation region by the computer program product controlling the sensor unit.

19. The computer program product according to any of claims 15 to 18, wherein the computer program product is configured to compare each image captured by an optical sensor with an irradiation region excluded from irradiation.

20. The computer program product according to any of claims 15 to 19, wherein the computer program product is configured to enter an operating mode by means of a signal from the sensor unit, in particular to search for a suitable irradiation region that is not excluded from irradiation.

21. The computer program product according to any of claims 15 to 20, wherein the computer program product causes exposure of an irradiation region to light radiation having wavelengths in a range of between 280 nm and 315 nm as soon as a sensor unit detects an irradiation region excluded from irradiation in the effective range of the lighting means.

22. The computer program product according to any of claims 15 to 21, which is further configured to monitor or configure the execution of control of a device for stimulating the biosynthesis of vitamin D₃ on a third-party apparatus by means of a communication unit.

23. The computer program product, which can be executed on a third-party apparatus and is configured to manage user data for a device according to any of claims 1 to 14,
a. wherein the user data contains data selected from the group consisting of: skin type, age, profession, and lifestyle, and
b. wherein the computer program product is configured to exchange said user data in encrypted form with a communication unit of the device.

## Revendications

1. Dispositif (1) pour stimuler la biosynthèse de la vitamine D₃, comprenant :
a. au moins un premier moyen d'éclairage (21) pour émettre un rayonnement lumineux comprenant des longueurs d'onde dans une plage comprise entre 280 nm et 315 nm ;
c. une unité d'orientation pour aligner l'au moins moyen d'éclairage vers la zone d'irradiation, et
d. une unité de commande qui est conçue pour déclencher une émission de rayonnement lumineux comprenant des longueurs d'onde dans une plage comprise entre 280 nm et 315 nm par l'au moins un moyen d'éclairage,
**caractérisé par**
b. une unité formant capteur pour reconnaître une zone d'irradiation ;
dans lequel l'unité de commande est en outre conçue pour identifier la zone d'irradiation.

2. Dispositif selon la revendication 1, dans lequel l'unité formant capteur comprend un capteur optique, en particulier une caméra.

3. Dispositif selon l'une des revendications 1 ou 2, dans lequel l'unité de commande est conçue pour commander un alignement du premier moyen d'éclairage par l'unité d'orientation en fonction d'une zone d'irradiation identifiée.

4. Dispositif selon l'une des revendications 1 à 3, comprenant une unité collectrice pour générer un faisceau dirigé de rayons à partir de l'émission de rayonnement lumineux par le premier moyen d'éclairage.

5. Dispositif selon la revendication 4, dans lequel l'unité collectrice comprend une lentille et/ou un collimateur, en particulier un collimateur avec une lentille collectrice pour générer un faisceau dirigé de rayons à partir de l'émission de rayonnement lumineux par le premier moyen d'éclairage.

6. Dispositif selon l'une des revendications 1 à 5, comprenant un deuxième moyen d'éclairage pour émettre un rayonnement lumineux comprenant des longueurs d'onde dans le domaine visible, en particulier comprenant des longueurs d'onde dans une plage comprise entre 380 nm et 720 nm.

7. Dispositif selon l'une des revendications 1 à 6, comprenant un troisième moyen d'éclairage pour émettre un rayonnement lumineux comprenant des longueurs d'onde dans le domaine du proche infrarouge, en particulier dans une plage comprise entre 720 nm et 1800 nm.

8. Dispositif selon l'une des revendications 1 à 7, dans lequel l'unité de commande est conçue pour identifier un visage humain dans une zone d'irradiation.

9. Dispositif selon l'une des revendications 1 à 8, dans lequel l'unité de commande est conçue pour identifier une zone d'irradiation prédéfinie.

10. Dispositif selon l'une des revendications 1 à 9, comprenant une unité de communication pour échanger des données.

11. Dispositif selon l'une des revendications 1 à 10, dans lequel le premier moyen d'éclairage et/ou le deuxième moyen d'éclairage et/ou le troisième moyen d'éclairage et l'unité formant capteur sont physiquement couplés de manière à pouvoir être orientés ensemble vers la zone d'irradiation par l'unité d'orientation.

12. Dispositif selon l'une des revendications 1 à 11, dans lequel l'unité d'orientation est conçue pour pouvoir pivoter sur au moins deux axes, en particulier par l'intermédiaire d'au moins un pied de support monté rotatif et un bras d'outil monté rotatif pour faire pivoter un moyen d'éclairage sur au moins deux axes.

13. Dispositif selon l'une des revendications 1 à 12, dans lequel la zone d'irradiation est une zone cutanée d'une superficie comprise entre 40 cm² et 900 cm², en particulier entre 50 cm² et 600 cm², en particulier environ 400 cm².

14. Dispositif selon la revendication 13, dans lequel la zone cutanée peut être définie par une unité collectrice réglable.

15. Produit programme informatique pour la réalisation d'une commande d'un dispositif comprenant une unité formant capteur et un premier moyen d'éclairage pour la stimulation de la biosynthèse de la vitamine D₃, en particulier un dispositif selon l'une des revendications 1 à 13, où
a. le produit programme informatique est conçu pour comparer une zone d'irradiation reconnue par ladite unité formant capteur avec des zones d'irradiation prédéfinies, et
b. le produit programme informatique est conçu pour déclencher une émission de rayonnement lumineux comprenant des longueurs d'onde dans une plage comprise entre 280 nm et 315 nm par ledit au moins un moyen d'éclairage lorsque la zone d'irradiation reconnue ne correspond pas à une zone d'irradiation exclue d'une irradiation.

16. Produit programme informatique selon la revendication 15, dans lequel une zone d'irradiation exclue d'une irradiation est une zone d'irradiation choisie dans le groupe consistant en : un visage humain, une zone cutanée avec un ou plusieurs naevi pigmentaires, une zone de peau couverte de croûtes, de plaies et/ou de cicatrices, et une zone cutanée déjà irradiée avec un rayonnement lumineux comprenant des longueurs d'onde dans une plage comprise entre 280 nm et 315 nm dans un intervalle de temps prédéfini.

17. Produit-programme informatique selon l'une des revendications 15 ou 16, le produit-programme informatique étant conçu pour attribuer à une zone d'irradiation définie un intervalle de temps pendant lequel la zone d'irradiation doit être exposée avec un rayonnement lumineux comprenant des longueurs d'onde dans une plage comprise entre 280 nm et 315 nm.

18. Produit programme informatique selon l'une des revendications 15 à 17, le produit programme informatique étant conçu pour commander une recherche d'une zone d'irradiation prédéfinie, le produit programme informatique commandant l'unité formant capteur.

19. Produit programme informatique selon l'une des revendications 15 à 18, le produit programme informatique étant conçu pour comparer chaque image prise par un capteur optique avec une zone d'irradiation exclue d'une irradiation.

20. Produit programme informatique selon l'une des revendications 15 à 19, le produit programme informatique étant conçu pour enregistrer un mode de fonctionnement par un signal de l'unité formant capteur, en particulier pour rechercher une zone d'irradiation appropriée qui n'est pas exclue d'une irradiation.

21. Produit programme informatique selon l'une des revendications 15 à 19, le produit programme informatique provoquant une suspension d'une irradiation d'une zone d'irradiation avec un rayonnement lumineux comprenant des longueurs d'onde dans une plage comprise entre 280 nm et 315 nm, dès qu'une unité formant capteur reconnaît une zone d'irradiation exclue d'une irradiation dans la zone effective du moyen d'éclairage.

22. Produit programme informatique selon l'une des revendications 15 à 21, conçu en outre pour surveiller ou configurer la réalisation d'une commande d'un dispositif pour stimuler la biosynthèse de la vitamine D₃ sur un appareil tiers par l'intermédiaire d'une unité de communication.

23. Produit programme informatique exécutable sur un appareil tiers et conçu pour gérer des données utilisateur pour un dispositif selon l'une des revendications 1 à 14,
a. dans lequel les données utilisateur comprennent des données choisies dans le groupe consistant en : type de peau, âge, profession et mode de vie, et
b. le produit programme informatique étant conçu pour échanger lesdites données utilisateur sous forme cryptée avec une unité de communication du dispositif.
